# EUROPEAN PATENT SPECIFICATION

(11) **EP 2 144 670 B1**
(45) Date of publication and mention of the grant of the patent: **11.06.2014**
(21) Application number: 07748076.2
(22) Date of filing: 27.04.2007
(51) Int. Cl.: A61N 1/365, A61N 1/368

(54) **IMPLANTABLE MEDICAL DEVICE FOR MONITORING VALVE MOVEMENTS OF A HEART**
IMPLANTIERBARES MEDIZINPRODUKT ZUR ÜBERWACHUNG VON HERZKLAPPENBEWEGUNGEN
DISPOSITIF MEDICAL IMPLANTABLE DE SURVEILLANCE DE MOUVEMENTS DE VALVULES CARDIAQUES

(43) Date of publication of application: 20.01.2010
(73) Proprietor: St. Jude Medical AB, 175 84 Järfälla (SE)
(72) Inventor: BJÖRLING, Anders, S-169 37 Solna (SE); TUVSTEDT, Cecilia, S-113 23 Stockholm (SE); NILSSON, Kenth, S-184 60 Åkersberga (SE); NOREN, Kjell, S-171 58 Solna (SE)
(74) Representative: Sjölander, Henrik
(86) International application number: PCT/SE2007/000411
(87) International publication number: WO 2008/133552

(56) References cited:
- EP-A2- 0 765 671
- EP-A2- 0 765 671
- WO-A1-02/00296
- WO-A1-02/00296
- WO-A1-99/30777
- WO-A1-99/30777
- WO-A1-2007/091244
- WO-A2-2005/018570
- WO-A2-2005/018570
- US-A1- 2004 127 944
- US-A1- 2004 147 968
- US-A1- 2005 182 447
- US-A1- 2006 271 117
- US-A1- 2006 271 117
- US-A1- 2007 066 905

## Description

### Technical field

The present invention generally relates to the field of implantable heart stimulation devices, such as pacemakers, implantable cardioverter-defibrillators (ICD), and similar cardiac stimulation devices that also are capable of monitoring and detecting electrical activities and events within the heart. More specifically, the present invention relates to an implantable medical device for monitoring the movements of the valve planes of the heart to determine at least one hemodynamic measure reflecting a mechanical functioning of a heart of a patient.

### Background art

Implantable heart stimulators that provide stimulation pulses to selected locations in the heart, e.g. selected chambers, have been developed for the treatment of cardiac diseases and dysfunctions. Heart stimulators have also been developed that affect the manner and degree to which the heart chambers contract during a cardiac cycle in order to promote the efficient pumping of blood.

Furthermore, the heart will pump more effectively when a coordinated contraction of both atria and ventricles can be provided. In a healthy heart, the coordinated contraction is provided through conduction pathways in both the atria and the ventricles that enable a very rapid conduction of electrical signals to contractile tissue throughout the myocardium to effectuate the atrial and ventricular contractions. If these conduction pathways do not function properly, a slight or severe delay in the propagation of electrical pulses may arise, causing asynchronous contraction of the ventricles which would greatly diminish the pumping efficiency of the heart. Patients who exhibit pathology of these conduction pathways, such as patients with bundle branch blocks, etc., can thus suffer from compromised pumping performance. For example, asynchronous movements of the valve planes of the right and left side of the heart, e.g. an asynchronous opening and/or closure of the aortic and pulmonary valves, is such an asynchrony that affects the pumping performance in a negative way. This may be caused by right bundle branch block (RBBB), left bundle branch block (LBBB), or A-V block. In a well functioning heart the left and right side of the heart contract more or less simultaneously starting with the contraction of the atria flushing down the blood through the valves separating the atria from the ventricles. In the right side of the heart through the tricuspid valve and in the left side of the heart through the mitral valve. Shortly after the atrial contraction the ventricles contract resulting in increasing blood pressure inside the ventricles that first closes the one way valves to the atria and after that forces the outflow valves to open. In the right side of the heart it is the pulmonary valves, that separates the right ventricle from the pulmonary artery that leads the blood to the lung, which is opened. In the left side of the heart the aortic valve separates the left ventricle from the aorta that transports blood to the whole body. The outflow valves, the pulmonary valve and aortic valve, open when the pressure inside the ventricle exceeds the pressure in the pulmonary artery and aorta, respectively. The ventricles are separated by the intraventricular elastic septum. Hence, for a well functioning heart a substantially synchronous operation of the left and right hand side of the heart, e.g. a synchronous opening and/or closure of the aortic and pulmonary, is of a high importance.

Various prior art procedures have been developed for addressing disorders related to asynchronous function of the heart. For instance, cardiac resynchronization therapy (CRT) can be used for effectuating synchronous atrial and/or ventricular contractions. Furthermore, cardiac stimulators may be provided that deliver stimulation pulses at several locations in the heart simultaneously, such as biventricular stimulators. The stimulation pulses could also be delivered to different locations with a selected delay in an attempt to optimize the hemodynamic performance, e.g. synchronize the closure of the aortic and pulmonary valves, in relation to the specific cardiac dysfunction present at the time of implant.

Information about the mechanical functioning of a heart can be obtained by means electrical signals produced by the heart. In a healthy heart the sinus node, situated in the right atrium, generates electrical signals which propagates throughout the heart and control its mechanical movement. Some medical conditions, however, affect the relationship between the electrical and mechanical activity of the heart and, therefore, measurements of the electrical activity only cannot be relied upon as indicative of the true status of the heart or as suitable for triggering stimulation of the heart.

Consequently, there is a need within the art of methods and devices for obtaining accurate and reliable signals reflecting different aspects of mechanical functioning of the heart.

Impedance measurements has been shown to provide reliable information regarding the mechanical functioning of the heart. Through the impedance measurements, blood volume changes are detectable. Blood has a higher conductivity (lower impedance) than myocardial tissue and lungs. The impedance-volume relationship is inverse; the more blood - the smaller impedance. In EP 1 561 489, for example, transvalvular impedance measurements are made between an atrium and a ventricle electrode of a implanted electro-catheter to provide information indicative of the mechanical state of the heart. The information is used to control the pacing rate of a rate responsive pacemaker. In particular, the impedance between across the tricuspid valve between the atrium and the ventricle of the right hand side of the heart is measured.

However, in order to be able to optimize the functioning of the heart it is of interest to obtain information that provide a more complete picture of the mechanical functioning and the pumping action of the heart and that provide accurate and reliable information of the mechanical functioning and the pumping action of the heart.

### Summary of the invention

An object of the present invention is to address the problem of obtaining information that reflects the mechanical functioning and the pumping action of the heart.

A further object of the present invention is to provide a device and method that automatically obtains information that reflects the mechanical functioning and the pumping action of the heart in an accurate and reliable way.

These and other objects of the present invention are achieved by a device and method as claimed in the independent claims. Further embodiments are defined in the dependent claims.

According to an aspect of the present disclosure, there is provided an implantable medical device for determining at least one hemodynamic measure reflecting a mechanical functioning of a heart of a patient including a pace pulse generator adapted to produce cardiac stimulating pacing pulses and being connectable to at least one medical lead for delivering the pulses to cardiac tissue of the heart. The implantable medical device comprises an impedance measuring circuit adapted to, during impedance measuring sessions, measure impedance signals between at least a first pair of electrodes of the at least one medical lead, the at least first pair including at least one electrode located in an atrium of the heart and at least one valve plane electrode located substantially at the level of a valve plane the heart, and between at least a second pair of electrodes of the at least one medical lead, the at least second pair including at least one electrode located in a ventricle of the heart and at least one valve plane electrode located substantially at the level of the valve plane, wherein impedances reflecting valve plane movements are obtained; and a hemodynamic parameter determining circuit adapted to determine at least one hemodynamic parameter based on the impedances, wherein the at least one hemodynamic parameter reflects the mechanical functioning of a heart.

According to a second aspect of the present disclosure, there is provided a method for determining at least one hemodynamic measure reflecting a mechanical functioning of a heart of a patient using an implantable medical device including a pace pulse generator adapted to produce cardiac stimulating pacing pulses and being connectable to at least one medical lead for delivering the pulses to cardiac tissue of the heart. The method comprises the steps of, during impedance measuring sessions, measuring impedance signals between at least a first pair of electrodes of the at least one medical lead, the at least first pair including at least one electrode located in an atrium of the heart and at least one valve plane electrode located substantially at the level of a valve plane the heart, and between at least a second pair of electrodes of the at least one medical lead, the at least second pair including at least one electrode located in a ventricle of the heart and at least one valve plane electrode located substantially at the level of the valve plane, wherein impedances reflecting valve plane movements are obtained; and determining at least one hemodynamic parameter based on the impedances, wherein the at least one hemodynamic parameter reflects the mechanical functioning of a heart.

According to a third aspect of the present disclosure, there is provided a computer readable medium comprising instructions for bringing a programmable device to perform steps of a method according to the second aspect of the present invention.

Thus, the present invention is based on the advantageous idea of monitoring valve movements using electrodes placed adjacent to or substantially at the level of the valve plane of the heart by measuring impedance variations between at least one electrode placed adjacent to or substantially at the level of the valve plane and at least one electrode attached in an atrium and at least one electrode attached in a ventricle, respectively. The valve plane movements are caused by the pumping action of the heart, i.e. by the increased and decreased volume of the ventricles, and by studying the valve plane movements the contraction pattern and mechanical functioning of the heart can be monitored. The measured impedances can, in turn, be used to determine hemodynamic parameters reflecting the mechanical functioning of the heart. Thereby, it is possible to automatically obtain information that accurately and reliably reflects the mechanical functioning and the pumping action of the heart.

The obtained information regarding the mechanical functioning of the heart may, for example, be used to optimize parameters of the implantable medical device such as the AV or VV delay. In one embodiment of the present invention, the implantable medical comprises an AV and/or VV delay determining circuit adapted to initiate an optimization procedure, wherein the pace pulse generator is controlled to, based on the hemodynamic parameter, iteratively adjust a present AV and/or VV delay to optimize an AV and/or VV delay with respect to the hemodynamic parameter. Thereby, the AV and/or VV delay can be dynamically and automatically adjusted with respect to the present pumping action of the heart. Further, the adjustments of the AV and/or VV delay can be made dynamically as a response to a changing mechanical functioning of the heart.

In a further embodiment of the present disclosure, the impedance measuring circuit is adapted to, during impedance measuring sessions, measure impedance signals between the at least first pair of electrodes of the at least one medical lead including an electrode located in an atrium of the heart and at least one first valve plane electrode located substantially at the level of the valve plane in close proximity to the right atrium of the heart and at least one second valve plane electrode located substantially at the level of the valve plane in close proximity to the left atrium of the heart, respectively, and between the at least second pair of electrodes of the at least one medical lead including an electrode located in a ventricle of the heart and the valve plane electrodes located substantially at the level of the valve plane, respectively, wherein impedance signals reflecting valve plane movements at respective sides of the heart are obtained; and wherein the hemodynamic parameter determining circuit is adapted to determine a synchronicity measure based on the impedances, the synchronicity measure reflecting a synchronicity between the valve plane movements of the right hand side and the left hand side of the heart, respectively, during the measurement sessions. In embodiments of the present invention, a synchronicity between a closure of the aortic valve and the pulmonary valve and/or an opening of the aortic valve and the pulmonary valve are determined.

Thereby, it is possible to monitor the parallelity or synchronicity of the left and right hand side of the heart in an accurate and reliable way as well as the operation of the aortic valve and the pulmonary valves. In a well functioning heart the left and right side of the heart contract more or less simultaneously starting with the contraction of the atria flushing down the blood through the valves separating the atria from the ventricles. In the right side of the heart through the tricuspid valve and in the left side of the heart through the mitral valve. Shortly after the atrial contraction the ventricles contract resulting in increasing blood pressure inside the ventricles that first closes the one way valves to the atria and after that forces the outflow valves to open. In the right side of the heart it is the pulmonary valves, that separates the right ventricle from the pulmonary artery that leads the blood to the lung, which is opened. In the left side of the heart the aortic valve separates the left ventricle from the aorta that transports blood to the whole body. The outflow valves, the pulmonary valve and aortic valve, open when the pressure inside the ventricle exceeds the pressure in the pulmonary artery and aorta, respectively. Hence, for a well functioning heart a substantially synchronous opening and/or closure of the aortic and pulmonary is of a high importance.

In another embodiment of the present disclosure, a synchronicity between a closure of the mitral valve and the tricuspid valve, respectively, is determined based on the impedances.

According to a further example of the present disclosure, the AV and/or VV delay determining circuit adapted to initiate an optimization procedure, wherein the pace pulse generator is controlled to, based on the synchronicity measure, iteratively adjust a present AV and/or VV delay to identify an AV and/or VV delay that causes substantially synchronized valve plane movements of the right hand side and the left hand side of the heart, respectively, during a cardiac cycle.

In yet another example of the present disclosure, the AV and/or VV delay determining circuit is adapted to initiate an optimization procedure, wherein the pace pulse generator is controlled to, based on the synchronicity between a closure of the aortic valve and the pulmonary valve and/or the synchronicity between an opening of the aortic valve and the pulmonary valve, iteratively adjust a present AV and/or VV delay to identify an AV and/or VV delay that causes a substantially synchronized closure and/or opening of the aortic valve and the pulmonary valves.

Moreover, the AV and/or VV delay determining circuit may be adapted to initiate an optimization procedure, wherein the pace pulse generator is controlled to, based on the synchronicity between a closure of the mitral valve and the tricuspid valve, iteratively adjust a present AV and/or VV delay to identify an AV and/or VV delay that causes a substantially synchronized closure of the mitral and tricuspid valves.

In embodiments of the present disclosure, the impedance measuring circuit is adapted to determine a maximum and/or minimum impedance of each respective impedance for each cardiac cycle. Moreover, the impedance measuring circuit may be adapted to determine a maximum absolute derivative of each respective impedance for each cardiac cycle.

In still another example, the impedance measuring circuit is adapted to perform the impedance measuring sessions during successive cardiac cycles, wherein impedance signals reflecting valve plane movements during the successive cardiac cycles are obtained.

According to embodiments of the present disclosure, the at least one valve plane electrode is placed endocardially.

Alternatively, the at least one valve plane electrode is placed epicardially.

In certain embodiments of the present disclosure, the at least one valve plane electrode is placed intrapericardially on the surface of the heart.

According to further examples of the present disclosure, the first valve plane electrode is placed endocardially in the right atrium, or in the left atrium, or in the left ventricle, or in the right ventricle, or epicardially and the second valve plane electrode is placed endocardially in the right atrium, or in the left atrium, or in the left ventricle, or in the right ventricle or epicardially.

As the skilled person realizes, steps of the methods, as well as preferred embodiments thereof, are suitable to realize as computer program or as a computer readable medium.

Further objects and advantages of the present invention will be discussed below by means of exemplifying embodiments.

### Brief description of the drawings

Exemplifying embodiments of the invention will be described below with reference to the accompanying drawings, in which:
Fig. 1 is a simplified partly cutaway view illustrating an implantable stimulator including an electrode configuration;
Fig. 2 is a simplified partly cutaway view illustrating an electrode configuration;
Fig. 3 is a simplified partly cutaway view illustrating an electrode configuration;
Fig. 4 is a simplified partly cutaway view illustrating an electrode configuration;
Fig. 5 is a simplified partly cutaway view illustrating an electrode configuration;
Fig. 6 is a simplified partly cutaway view illustrating an electrode configuration;
Fig. 7 is a simplified partly cutaway view illustrating an electrode configuration;
Fig. 8 is a simplified partly cutaway view illustrating an electrode configuration;
Fig. 9 is a simplified partly cutaway view illustrating an electrode configuration;
Fig. 10 is a simplified partly cutaway view illustrating an electrode configuration;
Fig. 11 is an illustration in a block diagram form of an implantable stimulator according to the embodiment shown in Fig. 1; and
Fig. 12 is a flow chart describing the principles of the present disclosure.

### Description of exemplifying embodiments

The following is a description of exemplifying embodiments. This description is not to be taken in limiting sense, but is made merely for the purposes of describing the general principles of the invention. Thus, even though particular types of implantable medical devices such as heart stimulators will be described, e.g. biventricular pacemakers, the invention is also applicable to other types of cardiac stimulators such as dual chamber stimulators, implantable cardioverter defibrillators (ICDs), etc.

In the following a number of different electrode configurations suitable for obtaining impedances reflecting the mechanical functioning of the heart, and in particular movements of the valve plane, will be discussed.

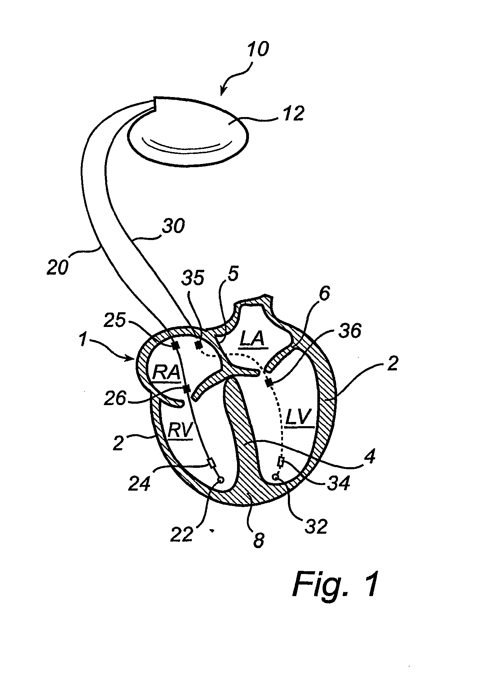

With reference first to Fig. 1, there is shown a implantable medical device. The invention is implemented in a stimulation device 10. The stimulation device 10 is in electrical communication with a patient's heart 1 by way of two leads 20 and 30 suitable for delivering multi-chamber stimulation, which leads 20 and 30 are connectable to the stimulator 10. The illustrated portions of the heart 1 include right atrium RA, the right ventricle RV, the left atrium LA, the left ventricle LV, cardiac walls 2, the ventricle septum 4, the valve plane 6, and the apex 8. The valve plane 6 refers to the annulus fibrosis plane separating the ventricle from the atria and containing all four heart valves, i.e. the aortic, pulmonary, mitral, and tricuspid valves.

In order to sense right ventricular and atrium cardiac signals and impedances and to provide stimulation therapy to the right ventricle RV, the stimulation device 10 is coupled to an implantable right ventricular lead 20 having a ventricular tip electrode 22, a ventricular annular or ring electrode 24, and a first valve plane electrode 26. The ring electrode 24 is arranged for sensing electrical activity, intrinsic or evoked, in the right ventricle RV. The right ventricular tip electrode 22 is arranged to be implanted in the endocardium of the right ventricle, e.g. near the apex 8 of the heart. Thereby, the tip electrode 22 becomes attached to cardiac wall. In this example, the tip electrode 22 is fixedly mounted in a distal header portion of the lead 20. Furthermore, the first valve plane electrode 26, which may a annular or ring electrode, is located substantially at the level of the valve plane 6.

In order to sense left atrium and ventricular cardiac signals and impedances and to provide pacing therapy for the left ventricle LV, the stimulation device 10 is coupled to a "coronary sinus" lead 30 designed for placement via the coronary sinus in veins located distally thereof, so as to place a distal electrode adjacent to the left ventricle and an electrode adjacent to the right atrium RA. The coronary sinus lead 30 is designed to received ventricular cardiac signals from the cardiac stimulator 10 and to deliver left ventricular LV pacing therapy using at least a left ventricular tip electrode 32 to the heart 1. In the illustrated example, the LV lead 30 comprises an annular ring electrode 34 for sensing electrical activity related to the left ventricle LV of the heart. Moreover, a second valve plane electrode 36, which may a annular or ring electrode, is located substantially at the level of the valve plane 6 and measurement electrode 35, which may a annular or ring electrode, is located adjacent to the right atrium RA.

With reference to Fig. 1, the impedances that can be detected by means of the illustrated embodiment will be described. At the right side of the heart 1, the impedance Z₂₆₋₂₂ between right ventricular tip electrode 22 and the valve plane electrode 26 and the impedance Z₂₆₋₃₅ between the valve plane electrode 26 and the electrode 35 located adjacent to the right atrium RA can be detected, respectively. Furthermore, at the left hand side of the heart 1, the impedance Z₃₆₋₃₂ between the left ventricular tip electrode 32 and the valve plane electrode 36 and the impedance 2₃₆₋₃₅ between the valve plane electrode 36 and the electrode 35 located adjacent to the right atrium RA can be detected, respectively. Since the electrode 35 located adjacent to the right atrium RA and the ventricle electrodes 22 and 32 essentially do not move during the cardiac cycle, the variation in the impedances are mainly due to movements of the valve plane 6.

Thus, the impedance Z₂₆₋₃₅ and the impedance Z₂₆₋₂₂, respectively, will vary during the cardiac cycle as a response to the movements of the valve plane 6 at the right hand side of the heart 1. Similarly, at the left hand side of the heart 1, the impedance Z₃₆₋₃₅ the impedance Z₃₆₋₃₂ will vary during the cardiac cycle as a response to the movements of the valve plane 6.

Moreover, the impedance Z₃₆₋₂₂ will be substantially constant over the cardiac cycle, which also is the case for the impedance Z₃₆₋₃₂. By comparing the detected impedances of the respective sides of the heart, asynchronicity or parallelity of the valve plane movements of the respective sides of the valve plane 6 can be determined. In case of an asynchronous depolarization sequence of the heart, the valve plane may move asynchronously and be bent during the heart cycle which will be reflected by an asynchronicity between the detected impedance at the right hand side and the left hand side, respectively.

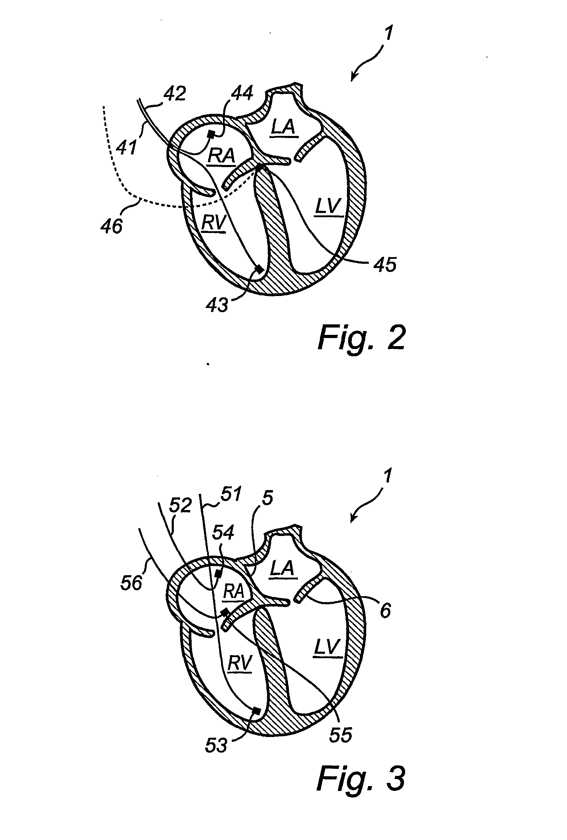

Turning briefly to Figs. 2-9, alternative embodiments for placement of cardiac leads, and cardiac electrodes are illustrated. In Fig. 2, an embodiment including two endocardially positioned leads 41 and 42 connected to a stimulation device (see Fig. 1) comprising an atrial distal tip electrode 44 located in the right atrium RA and a ventricular distal tip electrode 43 located in the right ventricle RV, respectively. The leads 41 and 42 can be fixedly attached to the cardiac wall according to conventional practice. Thereby, the electrodes 43 and 44 will be essentially immobile during the cardiac cycle. A third electrode 45 is located epicardially and is connected to the stimulation device by means of a lead 46. The lead 46 and the electrode 45 can be located epicardially by means of, for example, intrapercardial implantation technique. The electrode 45 is placed at the level of the valve plane 6. Thereby, the variation in the impedance Z₄₅₋₄₃ between the electrode 45 placed at the level of the valve plane 6 and the electrode 43 placed in the right ventricle RV and the impedance Z₄₅₋₄₄ between the electrode 45 placed at the level of the valve plane 6 and the electrode 44 placed in the right atrium RA, respectively, are mainly due to movements of the valve plane. The impedance Z₄₄₋₄₃ between the electrode 44 placed in the right atrium RA and the electrode 43 placed in the right ventricle RV will essentially by the same over a cardiac cycle since the electrodes 43, 44 are essentially immobile during the cardiac cycle.

With reference now to Fig. 3, an embodiment in which the impedances are measured by means of three endocardially placed electrodes. Similar to the embodiment shown in Fig. 2, two endocardially positioned leads 51 and 52 connected to a stimulation device (see Fig. 1) comprising an atrial distal tip electrode 54 located in the right atrium RA and a ventricular distal tip electrode 53 located in the right ventricle RV, respectively. The leads 51 and 52 can be fixedly attached to the cardiac wall according to conventional practice. Thereby, the electrodes 53 and 54 will be essentially immobile during the cardiac cycle. The third electrode 55 is also located endocardially in the right atrium RA and is connected to the stimulation device by means of a lead 46, transvenously advanced through a vein to the inside of the heart 1. The electrode 55 is placed at the level of the valve plane 6. Thereby, the variation in the impedance Z₅₅₋₅₃ between the electrode 55 placed at the level of the valve plane 6 in the right atrium RA and the electrode 53 placed in the right ventricle RV and the impedance Z₅₅₋₅₄ between the electrode 55 placed at the level of the valve plane 6 in the right atrium RA and the electrode 54 placed in the upper part of the right atrium RA, respectively, is mainly due to movements of the valve plane 6. The impedance Z₅₄₋₅₃ between the electrode 54 placed in the upper part of the right atrium RA and the electrode 53 placed in the right ventricle RV will essentially be the same over a cardiac cycle since the electrodes 53, 54 are essentially immobile during the cardiac cycle.

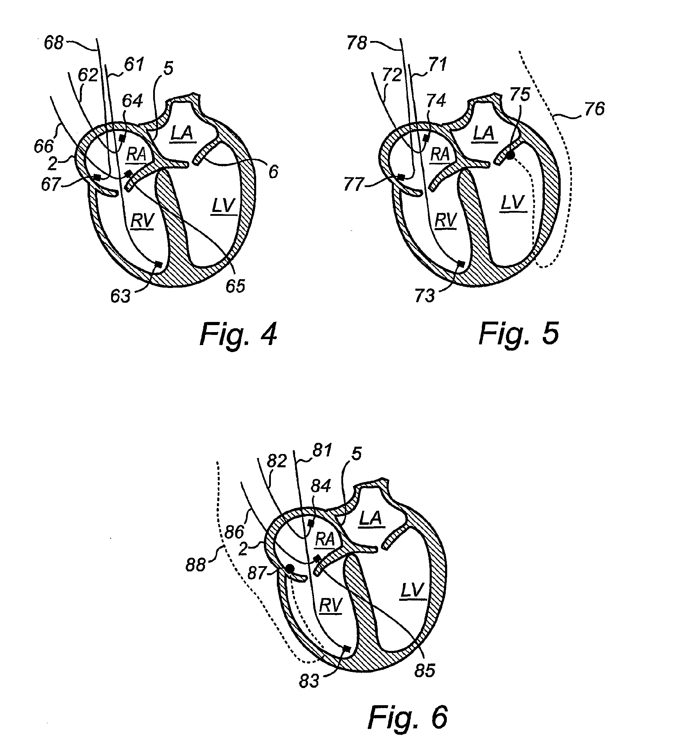

Referring now to Fig. 4, yet another electrode configuration for measuring impedances reflecting the valve plane movements will be described. According to this embodiment, two endocardially positioned leads 61 and 62 connected to a stimulation device (see Fig. 1) comprising an atrial distal tip electrode 64 located in the right atrium RA and a ventricular distal tip electrode 63 located in the right ventricle RV, respectively. The leads 61 and 62 can be fixedly attached to the cardiac wall according to conventional practice. Thereby, the electrodes 63 and 64 will be essentially immobile during the cardiac cycle. Further, two electrodes are placed at the level of the valve plane 6 endocardially in this configuration. A first valve plane electrode 65 is placed endocardially at the level of the valve plane 6 in the right atrium RA adjacent to atrial septum 5 and a second valve plane electrode 67 is placed endocardially at the level of the valve plane in the right atrium RA adjacent to the cardiac wall 2. The first and second valve plane electrodes 65, 67 are arranged at leads 66, 68 transvenously advanced through a vein to the inside of the heart 1 and connected to the stimulation device (see Fig. 1). The variations in the impedance Z₆₅₋₆₃ between the electrode 65 placed at the level of the valve plane 6 in the right atrium RA adjacent to the atrial septum 5 and the electrode 63 placed in the right ventricle RV and the impedance Z₆₅₋₆₄ between the electrode 65 placed at the level of the valve plane 6 in the right atrium RA adjacent to the atrial septum 5 and the electrode 64 placed in the upper part of the right atrium RA, respectively, are mainly due to movements of the valve plane 6. Similarly, the variations in the impedance Z₆₇₋₆₃ between the electrode 67 placed at the level of the valve plane 6 in the right atrium RA adjacent to the cardiac wall 2 and the electrode 63 placed in the right ventricle RV and the impedance Z₆₇₋₆₄ between the electrode 67 placed at the level of the valve plane 6 in the right atrium RA adjacent to the cardiac wall 2 and the electrode 64 placed in the upper part of the right atrium RA, respectively, are mainly due to movements of the valve plane 6. The impedance Z₆₄₋₆₃ between the electrode 64 placed in the upper part of the right atrium RA and the electrode 63 placed in the right ventricle RV will essentially by the same over a cardiac cycle since the electrodes 63, 64 are essentially immobile during the cardiac cycle.

In Fig. 5, a further embodiment is shown. Two leads 71 and 72 are endocardially positioned and connected to a stimulation device (see Fig. 1) comprising an atrial distal tip electrode 74 located in the right atrium RA and a ventricular distal tip electrode 73 located in the right ventricle RV, respectively. The leads 71 and 72 can be fixedly attached to the cardiac wall according to conventional practice. Thereby, the electrodes 73 and 74 will be essentially immobile during the cardiac cycle. Two electrodes are placed at the level of the valve plane 6 in this configuration. A first valve plane electrode 75 is placed epicardially at the level of the valve plane 6 at the left hand side of the heart 1 and a second valve plane electrode 77 is placed endocardially at the level of the valve plane in the right atrium RA adjacent to the cardiac wall 2. The first valve plane 75 is arranged on a lead 76 located epicardially by means of, for example, intrapercardial implantation technique, and connected to the stimulation device (see Fig. 1). The second valve plane electrode 77 is arranged at a lead 78 transvenously advanced through a vein to the inside of the heart 1 and connected to the stimulation device (see Fig. 1). The variations in the impedance Z₇₅₋₇₃ between the electrode 65 placed at the level of the valve plane 6 and the electrode 73 placed in the right ventricle RV and in the impedance Z₇₅₋₇₄ between the electrode 75 placed at the level of the valve plane 6 on the left hand side of the heart 1 and the electrode 74 placed in the upper part of the right atrium RA, respectively, are mainly due to movements of the valve plane 6 on the left hand side of the heart 1. Similarly, the variations in the impedance Z₇₇₋₇₃ between the electrode 77 placed at the level of the valve plane 6 in the right atrium RA adjacent to the cardiac wall 2 and the electrode 73 placed in the right ventricle RV and the impedance Z₇₇₋₇₄ between the electrode 77 placed at the level of the valve plane 6 in the right atrium RA adjacent to the cardiac wall 2 and the electrode 74 placed in the upper part of the right atrium RA, respectively, are mainly due to movements of the valve plane 6 at the right hand side of the heart 1. By comparing the impedance Z₇₇₋₇₄ and the Z₇₇₋₇₃ it is possible to monitor and detect the parallelity or synchronism between the valve plane movements at the respective sides of the heart. The impedance Z₇₄₋₇₃ between the electrode 74 placed in the upper part of the right atrium RA and the electrode 73 placed in the right ventricle RV will essentially by the same over a cardiac cycle since the electrodes 73, 74 are essentially immobile during the cardiac cycle.

Turning to Fig. 6, a further embodiment will be described. Two leads 81 and 82 are endocardially positioned, as in the embodiment shown in Fig. 5, comprising an atrial distal tip electrode 84 located in the right atrium RA and a ventricular distal tip electrode 83 located in the right ventricle RV, respectively. The leads 81 and 82 can be fixedly attached to the cardiac wall according to conventional practice. Thereby, the electrodes 83 and 84 will be essentially immobile during the cardiac cycle. Further, a first valve plane electrode 85 is placed endocardially at the level of the valve plane in the right atrium RA adjacent to the atrial septum 5 and a second valve plane electrode 87 is placed epicardially at the level of the valve plane 6 adjacent to the cardiac wall 2. The first valve plane electrode 85 is arranged at a lead 86 transvenously advanced through a vein to the inside of the heart 1 and connected to the stimulation device (see Fig. 1). The second valve plane 87 is arranged on a lead 88 located epicardially by means of, for example, intrapercardial implantation technique, and connected to the stimulation device (see Fig. 1). The electrode configurations of this embodiment is similar to the electrode configurations shown in Fig. 4 and thus the measured impedances will be similar to the impedances measured using the configuration shown in Fig. 4 for what reason a detailed description thereof is omitted.

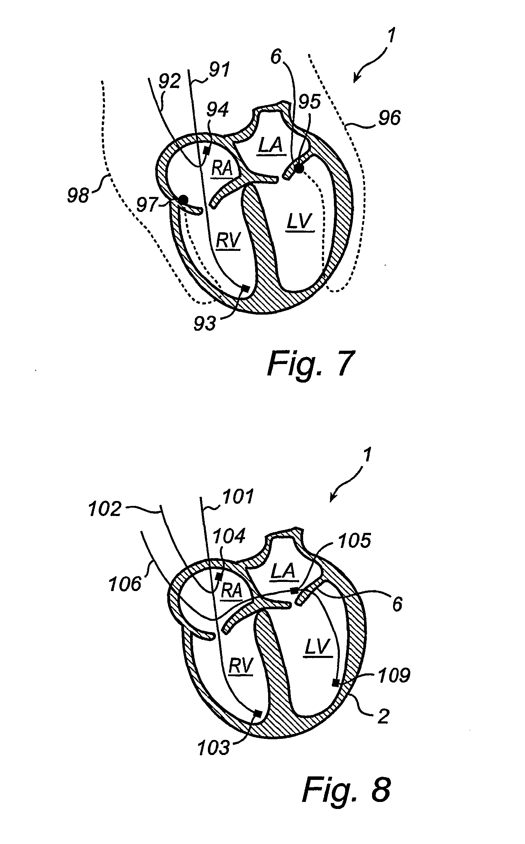

In Fig. 7, another configuration of electrodes for measuring the valve plane movements by means of impedances will be shown. As can be seen, two leads 91 and 92 are endocardially positioned, as in the embodiment shown in Fig. 5, comprising an atrial distal tip electrode 94 located in the right atrium RA and a ventricular distal tip electrode 93 located in the right ventricle RV, respectively. The leads 91 and 92 can be fixedly attached to the cardiac wall according to conventional practice. Thereby, the electrodes 93 and 94 will be essentially immobile during the cardiac cycle. Further, a first valve plane electrode 95 is placed epicardially at the level of the valve plane 6 at the left hand side of the heart 1 and a second valve plane electrode 97 is placed epicardially at the level of the valve plane 6 at the right hand side of the heart 1. The first valve plane electrode 95 and the second valve plane 97, respectively, are arranged on a leads 96, 98 located epicardially by means of, for example, intrapercardial implantation technique, and are connected to the stimulation device (see Fig. 1), respectively. he electrode configurations of this embodiment is similar to the electrode configurations shown in Fig. 5 and thus the measured impedances will be similar to the impedances measured using the configuration shown in Fig. 5 for what reason the description thereof is omitted.

Referring now to Fig. 8, yet another embodiment will be discussed. Two leads 101 and 92 are endocardially positioned comprising an atrial distal tip electrode 104 located in the right atrium RA and a ventricular distal tip electrode 103 located in the right ventricle RV, respectively. The leads 101 and 102 can be fixedly attached to the cardiac wall according to conventional practice. Thereby, the electrodes 103 and 104 will be essentially immobile during the cardiac cycle. In addition, a left ventricle lead 106 is placed in a left lateral coronary vein, advanced from the right atrium RA through the coronary sinus. The left ventricle lead 106 comprises a left ventricle tip electrode 109 and a valve plane electrode 105, e.g. an annular or ring electrode, located adjacent to the valve plane 6. The left ventricle lead 106 may be fixated at the cardiac wall using conventional practice. In this configuration, the valve plane movements detected will be substantially the valve plane movements at the left hand side of the heart 1. The impedance Z₁₀₅₋₁₀₃ between the electrode 105 placed at the level of the valve plane 6 and the electrode 103 placed in the right ventricle RV and in the impedance Z₁₀₅₋₁₀₄ between the electrode 105 placed at the level of the valve plane 6 on the left hand side of the heart 1 and the electrode 104 placed in the upper part of the right atrium RA, respectively, are measured and the variations are mainly due to movements of the valve plane 6 on the left hand side of the heart 1. Alternatively, the impedances Z₁₀₅₋₁₀₉ between the electrode 105 placed at the level of the valve plane 6 and the left ventricle electrode 109 and the impedance Z₁₀₅₋₁₀₃ between the electrode 105 placed at the level of the valve plane 6 and the electrode 103 placed in the right ventricle RV, respectively, can be measured.

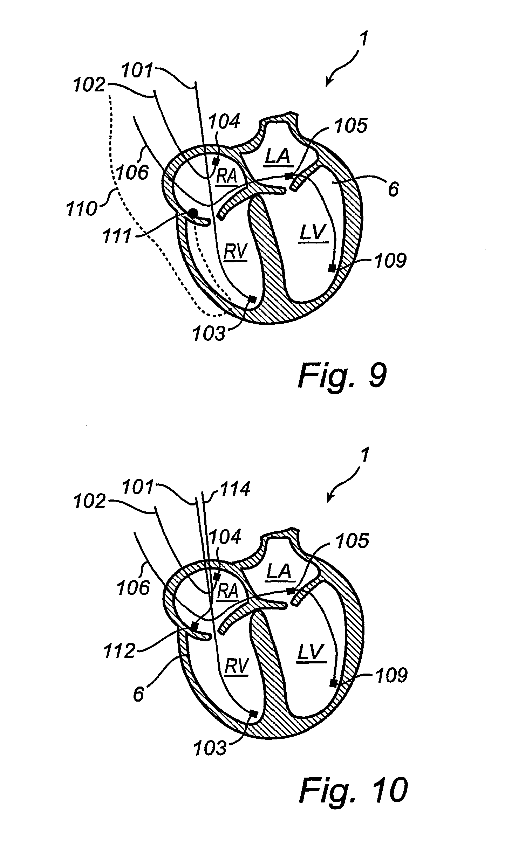

In Figs. 9 and 10, alternative electrode configurations to the configuration illustrated in Fig. 8 are shown. As can be seen in Fig. 8, a further lead 110 including a valve plane electrode 111 is located epicardially at the right hand side of the heart 1 by means of, for example, intrapercardial implantation technique, and connected to the stimulation device (see Fig. 1). Thereby, the impedances can be measured in a similar way as in the embodiment described with reference to Fig. 5 and it is possible to monitor and detect the parallelity or synchronism between the valve plane movements at the respective sides of the heart 1. In the embodiment shown in Fig. 9, a further lead 114 is transvenously advanced through a vein to the inside of the heart 1 and connected to the stimulation device (see Fig. 1), which lead 114 includes a second valve plane electrode 112 placed endocardially at the level of the valve plane in the right atrium RA adjacent to the cardiac wall 2. Thereby, the impedances can be measured in a similar way as in the embodiment described with reference to Fig. 5 and it is possible to monitor and detect the parallelity or synchronism between the valve plane movements at the respective sides of the heart 1. Thereby, the impedances can be measured in a similar way as in the embodiment described with reference to Fig. 5 and it is possible to monitor and detect the parallelity or synchronism between the valve plane movements at the respective sides of the heart 1.

Even though a number of examples have been illustrated in Figs. 1-10, the invention is not restricted the illustrated lead and electrode configurations and placements. For example, more epicardial electrodes can be placed at the level of the valve plane. Furthermore, different types of sensors can also be arranged in the leads to obtain other types of information. For example, cardiac wall motion sensors of accelerometer type can be arranged in a right ventricle and/or a left ventricle lead.

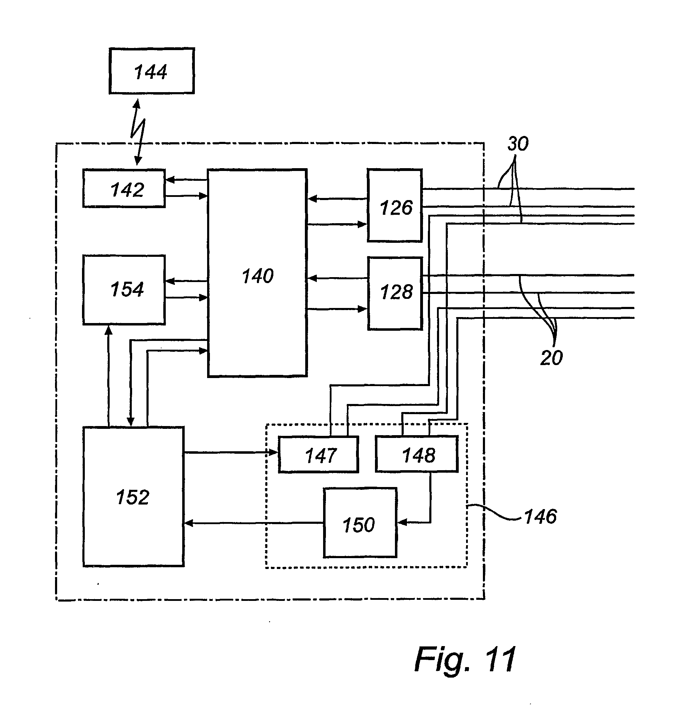

Turning now to Fig. 11, the heart stimulator 10 of Fig. 1 is shown in a block diagram form. For illustrative purposes, reference is made to Fig. 1 for the elements of the leads that are intended for positioning in or at the heart. The heart stimulator 10 is connected to a heart 1 order to sense heart signals and emit stimulation pulses to the heart 1. Electrodes located within and at the heart, for example, any one of the elctrode configurations illustrated in Figs. 1-10 and outside the heart, for example, an indifferent electrode 12 (which, in this instance, consists of the enclosure of the heart stimulator 10 but can also consist of a separate electrode located somewhere in the body) are connected to a pulse generator 126 in the heart stimulator 10. The electrodes located within and/or at the heart are connected to the stimulator 10 via leads, for example, the leads 20 and 30 shown in Fig. 1. A detector 128 is also connected to the electrodes in order to sense activity of the heart.

The pulse generator 126 and the detector 128 are controlled by a control unit 140 which regulates the stimulation pulses with respect to amplitude, duration and stimulation interval, the sensitivity of the detector 128 etc.

A physician using an extracorporeal programmer 144 can, via a telemetry unit 142, communicate with the heart stimulator 10 and thereby obtain information on identified conditions and also reprogram the different functions of the heart stimulator 10.

Furthermore, the heart stimulator 10 comprises an impedance measuring circuit 146 adapted to, during impedance measuring sessions, measure impedance signals between at least a first pair of electrodes, which at least first pair includes at least one electrode located in an atrium of the heart and at least one valve plane electrode located substantially at the level of a valve plane the heart. Further, the impedance measuring circuit 146 is adapted to, during the impedance measuring sessions, measure impedance signals between at least a second pair of electrodes, which at least second pair includes at least one electrode located in at least one ventricle of the heart and at least one valve plane electrode located substantially at the level of the valve plane. In Figs. 1-10, a number of different electrode configurations by which impedance signals reflecting the valve plane movements can be obtained are shown. The impedance measuring circuit 146 comprises a current generating circuit 147 adapted to apply excitation current pulses between the respective electrode pairs and a measuring circuit 148 adapted to measure the resulting voltage over the respective electrode pairs and determined resulting impedance signals. An impedance signal processor 150 is connected to the measuring circuit 148 and is adapted to process the impedance signals to determine respective impedances over each measurement session for the respective electrode pairs. The impedance signal processor 150 may be adapted to determine maximum or minimum impedances over a cardiac cycle, for example, for respective atrium and/or respective ventricle and/or to determine maximum absolute derivative of the impedance for respective atrium and/or respective ventricle.

Moreover, the heart stimulator 10 comprises a hemodynamic parameter determining circuit 152 adapted to determine at least one hemodynamic parameter based on impedances received from the impedance measuring circuit 146. The hemodynamic parameter determining circuit 152 comprises a microprocessor, which may, for example, control the impedance measuring circuit 146 to, inter alia, initiate an impedance measuring session, the length and/or amplitude of the generated current pulses. The at least one hemodynamic parameter based on the measured impedances reflects the mechanical functioning of the heart. A number of different parameters may be extracted from the measured impedances and monitored including pre-ejection period, a contraction patter, mitral regurgitation, a synchronicity between the left and right hand sides of the heart, etc.

In one embodiment, the hemodynamic parameter determining circuit 152 is adapted to determine a synchronicity measure based on the impedances reflecting the synchronicity between the valve plane movements of the right hand side and the left hand side of the heart, respectively, during impedance measurement sessions. Through the impedance measurements, blood volume changes are detected. Blood has a higher conductivity (lower impedance) than myocardial tissue and lungs. The impedance-volume relationship is inverse; the more blood - the smaller impedance. Accordingly, the impedance will vary over the cardiac cycle in connection with the contraction and filling of the atria and ventricles, respectively, in, hence, in connection with the pressure variations during the cycle. For example, the ventricle volume is at a maximum level at the onset of the systolic phase of the ventricles, which corresponds to a minimum impedance measured over the ventricles, and the ventricle volume is at a minimum level at onset of diastolic phase of the ventricles, which corresponds to a maximum impedance measured over the ventricles.

In one embodiment, the synchronicity between a closure of the aortic valve and the pulmonary valve and/or a synchronicity between an opening of the aortic valve and the pulmonary valve is determined using the measured impedances. For example, if the electrode configuration illustrated in Fig. 5 is used, the impedance Z₇₅₋₇₃ between the electrode 75 placed at the level of the valve plane 6 and the electrode 73 placed in the right ventricle RV reflects the movements of the valve plane 6 on the left hand side of the heart 1 and the variations of the blood volume of the right ventricle RV. Similarly, the variations in the impedance Z₇₇₋₇₃ between the electrode 77 placed at the level of the valve plane 6 in the right atrium RA adjacent to the cardiac wall 2 and the electrode 73 placed in the right ventricle RV reflects the movements of the valve plane 6 at the right hand side of the heart 1 and the variations of the blood volume of the left ventricle LV. By comparing the impedance Z₇₅₋₇₃ and the impedance Z₇₇₋₇₃ it is possible to detect an asynchronism between the valve plane movements at the respective sides of the heart, for example, the opening and/or closure of the aortic and pulmonary valves. For example, peak amplitudes of the impedances, maximum absolute derivative, or morphology of the impedance curves over a cardiac cycle can be studied to detect such an asynchronism.

The heart stimulator 10 further comprises an AV and/or VV delay determining circuit 154 adapted to determine a AV and/or VV delay with respect to a determined hemodynamic parameter, for example, a synchronicity between an opening and/or a closure of the pulmonary and aortic valves. In one embodiment, the AV and/or VV delay determining circuit 154 is integrated in the control circuit 140. The AV and/or VV delay determining circuit 154 is adapted to initiate an optimization procedure (e.g. via the control circuit 140), wherein the pace pulse generator 126 is controlled to iteratively adjust a present AV and/or VV delay to optimize an AV and/or VV delay with respect to the determined hemodynamic parameter starting from the determined AV and/or VV delay. For example, if it is determined that the movements of the valve plane at the right hand side of the heart is ahead the movements of the left hand side in the cardiac cycle, the VV delay may be adjusted such that the left ventricle is stimulated first and vice versa. However, it is important that AV delay of the left side has a sufficient length, i.e. if the different between the right side and the left side is large there is a risk that the effective AV delay on the left side becomes too short. In such a case, the AV delay should also be lengthened.

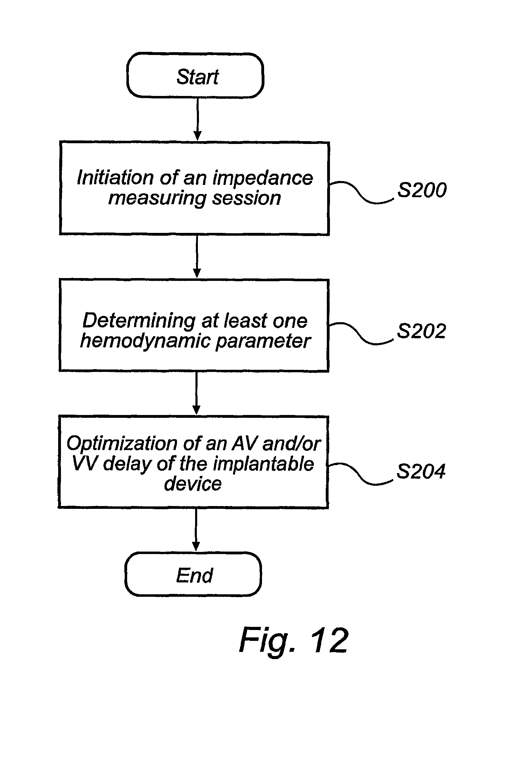

Turning now to Fig. 12, the principles of the present invention according to an embodiment will be described. First, at step 200, a request for an initiation of impedance measuring session is received by the hemodynamic parameter determining circuit 152. This request may be received from the control circuit 140 or from an external device via the telemetry unit 142. Alternatively, the hemodynamic parameter determining circuit 152 may be adapted to automatically initiate the impedance measuring sessions at regular intervals. The hemodynamic parameter determining circuit 152 sends an initiation signal to the impedance measuring circuit 146. This step may be preceded by a check of the measuring conditions, for example, which posture the patient is in, or the activity level. This measuring condition information may be used in the optimization of the AV and/or VV delay. Upon receipt of the initiation signal, which may include information regarding, for example, current pulse width or current amplitude, the impedance measuring circuit 146 measures, during impedance measuring sessions, impedances between at least a first pair of electrodes of the at least one medical lead, the at least first pair including at least one electrode located in an atrium of the heart and at least one valve plane electrode located substantially at the level of a valve plane the heart, and between at least a second pair of electrodes of the at least one medical lead, the at least second pair including at least one electrode located in a ventricle of the heart and at least one valve plane electrode located substantially at the level of the valve plane, wherein impedances reflecting valve plane movements are obtained, and in Figs. 1-10, a number of conceivable electrode configurations are illustrated. Thereafter, at step 202, at least one hemodynamic parameter based on the impedances, wherein the at least one hemodynamic parameter reflects the mechanical functioning of a heart, is determined. A synchronicity measure is determined, which reflects a parallelity or synchronicity between the left and right side of the heart. In particular, a parallelity or synchronicity between the valve planes at the left and right side of the heart can be monitored by means of the impedances, e.g. the synchronicity between an opening and/or closure of the aortic and pulmonary valves. Then, at step 204, an optimization procedure is initiated including adjusting present AV and/or VV delay to optimize an AV and/or VV delay with respect to the hemodynamic parameter.

In further embodiments, the indication of an asynchronicity between valve plane movements of the right and left side of the heart, for example, between the opening and/or closing of the aortic and pulmonary valves, could be used for triggering an alarm signal to the patient. This alarm signal could be intended for prompting the patient to seek medical assistance for care of follow-up. The alarm signal may alternatively, or as a compliment, be transferred to an extracorporeal unit 144 or a care institution via the telemetry unit 142.

While the invention disclosed herein has been described by means of specific embodiments and applications thereof, numerous modifications and variations could be made therein by those skilled in the art without departing from the scope of the inventions, which is defined by the appended claims.

## Claims

1. An implantable medical device (10) for determining at least one hemodynamic measure reflecting a mechanical functioning of a heart of a patient including a pace pulse generator (126) adapted to produce cardiac stimulating pacing pulses and being connectable to at least one medical lead (20, 30; 41, 42, 46; 51, 52, 56; 61, 62, 66, 68; 71, 72, 76, 78; 81, 82, 86, 88; 91, 92, 96, 98; 101, 102, 106, 110, 114) for delivering said pulses to cardiac tissue of said heart (1), comprising:
an impedance measuring circuit (146) adapted to, during impedance measuring sessions, measure impedance signals between at least a first pair of electrodes of said at least one medical lead (20, 30; 41, 42, 46; 51, 52, 56; 61, 62, 66, 68; 71, 72, 76, 78; 81, 82, 86, 88; 91, 92, 96, 98; 101, 102, 106, 110, 114), said at least first pair including at least one electrode (25, 35; 44; 54; 64; 74; 84; 94; 104) located in an atrium of said heart and at least one first valve plane electrode (26, 36; 45; 55; 65, 67; 75, 77; 85, 87; 95, 97; 105, 112, 111) located substantially at the level of a valve plane (6) said heart (1), and between at least a second pair of electrodes of said at least one medical lead (20, 30; 41, 42, 46; 51, 52, 56; 61, 62, 66, 68; 71, 72, 76, 78; 81, 82, 86, 88; 91, 92, 96, 98; 101, 102, 106, 110, 114), said at least second pair including at least one electrode (22, 24, 32, 34; 43; 53; 63; 73; 93; 103, 109) located in a ventricle of said heart and the at least one first valve plane electrode (26, 36; 45; 55; 65, 67; 75, 77; 85, 87; 95, 97; 105, 111, 112) located substantially at the level of said valve plane (6), wherein impedances reflecting valve plane movements of the respective sides of the heart are obtained using said at least one first valve plane electrode and at least one second valve plane electrode that is configured to be positioned on the other side of the heart than the at least one first valve plane electrode; and
a hemodynamic parameter determining circuit (152) adapted to determine at least one hemodynamic parameter based on comparisons between said impedances of the respective sides of the heart, wherein said at least one hemodynamic parameter reflects said mechanical functioning of the heart including a parallelity of the valve plane movements of the respective sides of said heart.

2. The implantable medical device according to claim 1, further comprising an AV and/or VV delay determining circuit (154) adapted to initiate an optimization procedure, wherein said pace pulse generator (126) is controlled to, based on said hemodynamic parameter, iteratively adjust a present AV and/or W delay to optimize an AV and/or W delay with respect to said hemodynamic parameter.

3. The implantable medical device according to claim 1 or 2,
wherein said impedance measuring circuit (146) adapted to, during impedance measuring sessions, measure impedance signals between said at least first pair of electrodes of said at least one medical lead (20, 30; 41, 42, 46; 51, 52, 56; 61, 62, 66, 68; 71, 72, 76, 78; 81, 82, 86, 88; 91, 92, 96, 98; 101, 102, 106, 110, 114) including an electrode (25, 35; 44; 54; 64; 74; 84; 94; 104) located in an atrium of said heart and at least one first valve plane electrode (26; 45; 55; 65, 67; 77; 85, 87; 97; 111, 112) located substantially at the level of said valve plane (6) in close proximity to the right atrium of said heart and at least one second valve plane electrode (36; 45; 75; 95; 105) located substantially at the level of said valve plane (6) in close proximity to the left atrium of said heart, respectively, and between said at least second pair of electrodes of said at least one medical lead (20, 30; 41, 42, 46; 51, 52, 56; 61, 62, 66, 68; 71, 72, 76, 78; 81, 82, 86, 88; 91, 92, 96, 98; 101, 102, 106, 110,114) including an electrode (22, 24, 32, 34; 43, 45; 53, 55; 63; 73; 93; 103, 109) located in a ventricle of said heart and said valve plane electrodes located substantially at the level of said valve plane (6), respectively, wherein impedance signals reflecting valve plane movements at respective sides of the heart are obtained; and
wherein said hemodynamic parameter determining circuit (152) is adapted to determine a synchronicity measure based on said impedances, said synchronicity measure reflecting a synchronicity between the valve plane movements of the right hand side and the left hand side of the heart, respectively, during said measurement sessions.

4. The implantable medical device according to claim 3, wherein said hemodynamic parameter determining circuit (152) is adapted to determine a synchronicity between a closure of the aortic valve and the pulmonary valve and/or to determine a synchronicity between an opening of the aortic valve and the pulmonary valve.

5. The implantable medical device according to claim 3 or 4, wherein said hemodynamic parameter determining circuit (152) is adapted to determine a synchronicity between a closure of the mitral valve and the tricuspid valve, respectively, based on said impedances.

6. The implantable medical device according to claim 3-5, wherein said AV and/or W delay determining circuit (adapted to
initiate an optimization procedure, wherein said pace pulse generator is controlled to, based on said synchronicity measure, iteratively adjust a present AV and/or W delay to identify an AV and/or VV delay that causes substantially synchronized valve plane movements of the right hand side and the left hand side of the heart, respectively, during a cardiac cycle.

7. The implantable medical device according to claim 6, wherein said an AV and/or W delay determining circuit (154) is adapted to
initiate an optimization procedure, wherein said pace pulse generator (126) is controlled to, based on said synchronicity between a closure of the aortic valve and the pulmonary valve and/or said synchronicity between an opening of the aortic valve and the pulmonary valve, iteratively adjust a present AV and/or W delay to identify an AV and/or VV delay that causes a substantially synchronized closure and/or opening of the aortic and the pulmonary valves.

8. The implantable medical device according to claim 6 or 7, wherein said an AV and/or W delay determining circuit (154) is adapted to
initiate an optimization procedure, wherein said pace pulse generator (126) is controlled to, based on said synchronicity between a closure of the mitral valve and the tricuspid valve, iteratively adjust a present AV and/or W delay to identify an AV and/or W delay that causes a substantially synchronized closure of the mitral and tricuspid valves.

9. The implantable medical device according to claim 1-8, wherein said impedance measuring circuit (146) is adapted to determine a maximum and/or minimum impedance of each respective impedance for each cardiac cycle.

10. The implantable medical device according to claim 1-9, wherein said impedance measuring circuit (146) is adapted to determine a maximum absolute derivative of each respective impedance for each cardiac cycle.

11. The implantable medical device according to claim 1-10, wherein
said impedance measuring circuit (146) is adapted to perform said impedance measuring sessions during successive cardiac cycles, wherein impedance signals reflecting valve plane movements during said successive cardiac cycles are obtained.

12. The implantable medical device according to claim 1-8, wherein said at least one valve plane electrode (26, 36; 55; 65, 67; 77; 85; 105, 112) is placed endocardially.

13. The implantable medical device according to claim 1-12, wherein said at least one valve plane electrode (45; 75; 87; 95, 97; 111) is placed epicardially.

14. The implantable medical device according to claim 2-13, wherein said first valve plane electrode (26, 36; 55; 65, 67; 77; 85; 105, 112) is placed endocardially in the right atrium, or in the left atrium, or in the left ventricle, or in the right ventricle, or epicardially and wherein said second valve plane electrode is placed endocardially (45; 75; 87; 95, 97; 111) in the right atrium, or in the left atrium, or in the left ventricle, or in the right ventricle or epicardially.

## Patentansprüche

1. Implantierbares Medizinprodukt (10) zum Bestimmen von mindestens einer hämodynamischen Größe, die eine mechanische Funktion eines Herzens eines Patienten anzeigt, das einen Stimulierungsimpulsgenerator (126) aufweist, der so ausgelegt ist, dass er Herzstimulationserregungsimpulse erzeugt, und mit mindestens einer medizinischen Leitung (20, 30; 41, 42, 46; 51, 52, 56; 61, 62, 66, 68; 71, 72, 76, 78; 81, 82, 86, 88; 91, 92, 96, 98; 101, 102, 106, 110, 114) zum Abgeben der Impulse an Herzgewebe des Herzens (1) verbindbar ist, umfassend:
eine Impedanzmessschaltung (146), die so ausgelegt ist, dass sie während Impedanzmessvorgängen Impedanzsignale misst zwischen mindestens einem ersten Elektrodenpaar der mindestens einen medizinischen Leitung (20, 30; 41, 42, 46; 51, 52, 56; 61, 62, 66, 68; 71, 72, 76, 78; 81, 82, 86, 88; 91, 92, 96, 98; 101, 102, 106, 110, 114); wobei das mindestens eine erste Paar mindestens eine Elektrode (25, 35; 44; 54; 64; 74; 84; 94; 104) aufweist, die in einem Vorhof des Herzens angeordnet ist, und mindestens eine erste Klappenebenen-Elektrode (26, 36; 45; 55; 65, 67; 75, 77; 85, 87; 95, 97; 105, 112, 111), die im Wesentlichen auf Höhe einer Klappenebene (6) des Herzens (1) angeordnet ist, und zwischen mindestens einem zweiten Elektrodenpaar der mindestens einen medizinischen Leitung (20, 30; 41, 42, 46; 51, 52, 56; 61, 62, 66, 68; 71, 72, 76, 78; 81, 82, 86, 88; 91, 92, 96, 98; 101, 102, 106, 110, 114), wobei das mindestens eine zweite Paar mindestens eine Elektrode (22, 24, 32, 34; 43; 53; 63; 73; 93; 103, 109) aufweist, die in einer Herzkammer des Herzens angeordnet ist, und die mindestens eine erste Klappenebenen-Elektrode (26, 36; 45; 55; 65, 67; 75, 77; 85, 87; 95, 97; 105, 111, 112), die im Wesentlichen auf Höhe der Klappenebene (6) angeordnet ist, wobei Impedanzen, die Klappenebenenbewegungen der jeweiligen Seiten des Herzens anzeigen, unter Verwendung der mindestens einen ersten Klappenebenen-Elektrode und mindestens einer zweiten Klappenebenen-Elektrode erhalten werden, die so eingerichtet ist, dass sie auf der anderen Seite des Herzens als die mindestens eine erste Klappenebenen-Elektrode platziert ist; und
eine Schaltung zur Bestimmung hämodynamischer Parameter (152), die so ausgelegt ist, dass sie mindestens einen hämodynamischen Parameter auf der Grundlage von Vergleichen zwischen den Impedanzen der jeweiligen Seiten des Herzens bestimmt, wobei der mindestens eine hämodynamische Parameter die mechanische Funktion des Herzens einschließlich einer Parallelität der Klappenebenenbewegungen der jeweiligen Seiten des Herzens anzeigt.

2. Implantierbares Medizinprodukt nach Anspruch 1, ferner umfassend eine Schaltung zur Bestimmung des AV- und/oder VV-Intervalls (154), die so ausgelegt ist, dass sie ein Optimierungsverfahren startet, wobei der Stimulierungsimpulsgenerator (126) so gesteuert ist, dass er auf der Grundlage des hämodynamischen Parameters iterativ ein vorliegendes AV- und/oder VV-Intervall anpasst, um ein AV- und/oder VV-Intervall hinsichtlich des hämodynamischen Parameters zu optimieren.

3. Implantierbares Medizinprodukt nach Anspruch 1 oder 2,
wobei die Impedanzmessschaltung (146) so ausgelegt ist, dass sie während Impedanzmessvorgängen Impedanzsignale misst zwischen dem mindestens einen ersten Elektrodenpaar der mindestens einen medizinischen Leitung (20, 30; 41, 42, 46; 51, 52, 56; 61, 62, 66, 68; 71, 72, 76, 78; 81, 82, 86, 88; 91, 92, 96, 98; 101, 102, 106, 110, 114), das eine Elektrode (25, 35; 44; 54; 64; 74; 84; 94; 104), die in einem Vorhof des Herzens angeordnet ist, und mindestens eine erste Klappenebenen-Elektrode (26; 45; 55; 65, 67; 77; 85, 87; 97; 111, 112), die im Wesentlichen auf Höhe der Klappenebene (6) sehr nahe am rechten Vorhof des Herzens angeordnet ist, beziehungsweise mindestens eine zweite Klappenebenen-Elektrode (36; 45; 75; 95; 105) aufweist, die im Wesentlichen auf Höhe der Klappenebene (6) sehr nah an dem linken Vorhof des Herzens angeordnet ist, und zwischen dem mindestens einen zweiten Elektrodenpaar der mindestens einen medizinischen Leitung (20, 30; 41, 42, 46; 51, 52, 56; 61, 62, 66, 68; 71, 72, 76, 78; 81, 82, 86, 88; 91, 92, 96, 98; 101, 102, 106, 110, 114), das eine Elektrode (22, 24, 32, 34; 43, 45; 53, 55; 63; 73; 93; 103, 109), die in einer Herzkammer des Herzens angeordnet ist, beziehungsweise die Klappenebenen-Elektroden aufweist, die im Wesentlichen auf Höhe der Klappenebene (6) angeordnet sind, wobei Impedanzsignale erhalten werden, die Klappenebenenbewegungen auf jeweiligen Seiten des Herzens anzeigen; und
wobei die Schaltung zur Bestimmung hämodynamischer Parameter (152) so ausgelegt ist, dass sie eine Synchronitätsgröße anhand der Impedanzen bestimmt, wobei die Synchronitätsgröße eine Synchronität zwischen den Klappenebenenbewegungen der rechten Seite beziehungsweise der linken Seite des Herzens während der Messvorgänge anzeigt.

4. Implantierbares Medizinprodukt nach Anspruch 3, wobei die Schaltung zur Bestimmung hämodynamischer Parameter (152) so ausgelegt ist, dass sie eine Synchronität zwischen dem Schließen der Aortenklappe und der Pulmonalklappe bestimmt und/oder eine Synchronität zwischen dem Öffnen der Aortenklappe und der Pulmonalklappe bestimmt.

5. Implantierbares Medizinprodukt nach Anspruch 3 oder 4, wobei die Schaltung zur Bestimmung hämodynamischer Parameter (152) so ausgelegt ist, dass sie eine Synchronität zwischen dem Schließen der Mitralklappe beziehungsweise der Trikuspidalklappe anhand der Impedanzen bestimmt.

6. Implantierbares Medizinprodukt nach Anspruch 3 bis 5, wobei die Schaltung zur Bestimmung des AV- und/oder VV-Intervalls so ausgelegt ist, dass sie
ein Optimierungsverfahren startet, wobei der Stimulierungsimpulsgenerator so gesteuert ist, dass er auf der Grundlage der Synchronitätsgröße iterativ ein vorliegendes AV- und/oder VV-Intervall anpasst, um ein AV- und/oder VV-Intervall zu ermitteln, das im Wesentlichen synchronisierte Klappenebenenbewegungen der rechten Seite beziehungsweise linken Seite des Herzens während eines Herzzyklus bewirkt.

7. Implantierbares Medizinprodukt nach Anspruch 6, wobei die Schaltung zur Bestimmung des AV- und/oder VV-Intervalls (154) so ausgelegt ist, dass sie
ein Optimierungsverfahren startet, wobei der Stimulierungsimpulsgenerator (126) so gesteuert ist, dass er auf der Grundlage der Synchronität zwischen dem Schließen der Aortenklappe und der Pulmonalklappe und/oder der Synchronität zwischen dem Öffnen der Aortenklappe und der Pulmonalklappe iterativ ein vorliegendes AV- und/oder VV-Intervall anpasst, um ein AV- und/oder VV-Intervall zu ermitteln, das ein im Wesentlichen synchronisiertes Schließen und/oder Öffnen der Aorten- und der Pulmonalklappe bewirkt.

8. Implantierbares Medizinprodukt nach Anspruch 6 oder 7, wobei die Schaltung zur Bestimmung des AV- und/oder W-Intervalls (154) so ausgelegt ist, dass sie
ein Optimierungsverfahren startet, wobei der Stimulierungsimpulsgenerator (126) so gesteuert ist, dass er auf der Grundlage der Synchronität zwischen dem Schließen der Mitralklappe und der Trikuspidalklappe iterativ ein vorliegendes AV- und/oder W-Intervall anpasst, um ein AV- und/oder W-Intervall zu ermitteln, das ein im Wesentlichen synchronisiertes Schließen der Mitral- und Trikuspidalklappe bewirkt.

9. Implantierbares Medizinprodukt nach Anspruch 1 bis 8, wobei die Impedanzmessschaltung (146) so ausgelegt ist, dass sie eine Höchst- und/oder Mindestimpedanz jeder jeweiligen Impedanz für jeden Herzzyklus bestimmt.

10. Implantierbares Medizinprodukt nach Anspruch 1 bis 9, wobei die Impedanzmessschaltung (146) so ausgelegt ist, dass sie eine maximale absolute Ableitung jeder jeweiligen Impedanz für jeden Herzzyklus bestimmt.

11. Implantierbares Medizinprodukt nach Anspruch 1 bis 10, wobei
die Impedanzmessschaltung (146) so ausgelegt ist, dass sie die Impedanzmessvorgänge während aufeinanderfolgender Herzzyklen durchführt, wobei Impedanzsignale, die Klappenebenenbewegungen während der aufeinanderfolgenden Herzzyklen anzeigen, erhalten werden.

12. Implantierbares Medizinprodukt nach Anspruch 1 bis 8, wobei die mindestens eine Klappenebenen-Elektrode (26, 36; 55; 65, 67; 77; 85; 105, 112) endokardial platziert ist.

13. Implantierbares Medizinprodukt nach Anspruch 1 bis 12, wobei die mindestens eine Klappenebenen-Elektrode (45; 75; 87; 95, 97; 111) epikardial platziert ist.

14. Implantierbares Medizinprodukt nach Anspruch 2 bis 13, wobei die erste Klappenebenen-Elektrode (26, 36; 55; 65, 67; 77; 85; 105, 112) endokardial im rechten Vorhof oder im linken Vorhof oder in der linken Herzkammer oder in der rechten Herzkammer oder epikardial platziert ist, und wobei die zweite Klappenebenen-Elektrode (45; 75; 87; 95, 97; 111) endokardial im rechten Vorhof oder im linken Vorhof oder in der linken Herzkammer oder in der rechten Herzkammer oder epikardial platziert ist.

## Revendications

1. Dispositif médical implantable (10) destiné à déterminer au moins une mesure hémodynamique reflétant un fonctionnement mécanique d'un coeur d'un patient, comportant un générateur d'impulsions de stimulation (126) adapté pour générer des impulsions de stimulation cardiaque et qui peut être raccordé à au moins une dérivation médicale (20, 30 ; 41, 42, 46 ; 51, 52, 56 ; 61, 62, 66, 66, 68 ; 71, 72, 76, 78 ; 81, 82, 86, 88 ; 91, 92, 96, 98 ; 101, 102, 106, 110, 114) pour transmettre lesdites impulsions à un tissu cardiaque dudit coeur (1), comprenant :
un circuit de mesure d'impédance (146) adapté pour, pendant des séances de mesure d'impédance, mesurer des signaux d'impédance entre au moins une première paire d'électrodes de ladite au moins une dérivation médicale (20, 30 ; 41, 42, 46 ; 51, 52, 56 ; 61, 62, 66, 68 ; 71, 72, 76, 78 ; 81, 82, 86, 88 ; 91, 92, 96, 98 ; 101, 102, 106, 110, 114) ; ladite au moins première paire comportant au moins une électrode (25, 35 ; 44 ; 54 ; 64 ; 74 ; 84 ; 94 ; 104) située dans une oreillette dudit coeur et au moins une première électrode de plan valvulaire (26, 36 ; 45 ; 55 ; 65, 67 ; 75, 77 ; 85, 87 ; 95, 97 ; 105, 112, 111) situé essentiellement au niveau d'un plan valvulaire (6) dudit coeur (1), et entre au moins une seconde paire d'électrodes de ladite au moins une dérivation médicale (20, 30 ; 41, 42, 46 ; 51, 52, 56 ; 61, 62, 66, 68 ; 71, 72, 76, 78 ; 81, 82, 86, 88 ; 91, 92, 96, 98 ; 101, 102, 106, 110, 114), ladite au moins seconde paire comportant au moins une électrode (22, 24, 32, 34 ; 43 ; 53 ; 63 ; 73 ; 93 ; 103, 109) située dans un ventricule dudit coeur et l'au moins une première électrode de plan valvulaire (26, 36 ; 45 ; 55 ; 65, 67 ; 75, 77 ; 85, 87 ; 95, 97 ; 105, 111, 112) située essentiellement au niveau dudit plan valvulaire (6), dans lequel des impédances reflétant des mouvements de plan valvulaire des côtés respectifs du coeur sont obtenues en utilisant ladite au moins une première électrode de plan valvulaire et au moins une seconde électrode de plan valvulaire qui est configurée pour être positionnée de l'autre côté du coeur que l'au moins une première électrode de plan valvulaire ; et
un circuit de détermination de paramètres hémodynamiques (152) adapté pour déterminer au moins un paramètre hémodynamique sur la base de comparaisons entre lesdites impédances des côtés respectifs du coeur, dans lequel ledit au moins un paramètre hémodynamique reflète ledit fonctionnement mécanique du coeur y compris une parallélité des mouvements de plan valvulaire des côtés respectifs dudit coeur.

2. Dispositif médical implantable selon la revendication 1, comprenant en outre un circuit de détermination de délai AV et/ou VV (154) adapté pour lancer une procédure d'optimisation, dans lequel ledit générateur d'impulsions de stimulation (126) est commandé pour, sur la base dudit paramètre hémodynamique, ajuster de manière itérative un délai AV et/ou VV présent pour optimiser un délai AV et/ou VV par rapport audit paramètre hémodynamique.

3. Dispositif médical implantable selon la revendication 1 ou 2,
dans lequel ledit circuit de mesure d'impédance (146) est adapté pour, pendant des séances de mesure d'impédance, mesurer des signaux d'impédance entre ladite au moins première paire d'électrodes de ladite au moins une dérivation médicale (20, 30 ; 41, 42, 46 ; 51, 52, 56 ; 61, 62, 66, 68 ; 71, 72, 76, 78 ; 81, 82, 86, 88 ; 91, 92, 96, 98 ; 101, 102, 106, 110, 114) comportant une électrode (25, 35 ; 44 ; 54 ; 64 ; 74 ; 84 ; 94 ; 104) située dans une oreillette dudit coeur et au moins une première électrode de plan valvulaire (26 ; 45 ; 55 ; 65, 67 ; 77 ; 85, 87 ; 97 ; 111, 112) située essentiellement au niveau dudit plan valvulaire (6) à proximité étroite de l'oreillette droite dudit coeur et respectivement au moins une seconde électrode de plan valvulaire (36 ; 45 ; 75 ; 95 ; 105) située essentiellement au niveau dudit plan valvulaire (6) à proximité étroite de l'oreillette gauche dudit coeur, et entre ladite au moins seconde paire d'électrodes de ladite au moins une dérivation médicale (20, 30 ; 41, 42, 46 ; 51, 52, 56 ; 61, 62, 66, 68 ; 71, 72, 76, 78 ; 81, 82, 86, 88 ; 91, 92, 96, 98 ; 101, 102, 106, 110, 114) comportant une électrode (22, 24, 32, 34 ; 43, 45 ; 53, 55 ; 63 ; 73 ; 93 ; 103, 109) située dans un ventricule dudit coeur et respectivement lesdites électrodes de plan valvulaire situées essentiellement au niveau dudit plan valvulaire (6), dans lequel des signaux d'impédance reflétant des mouvements de plan valvulaire à des côtés opposés du coeur sont obtenus ; et
dans lequel ledit circuit de détermination de paramètres hémodynamiques (152) est adapté pour déterminer une mesure de synchronisme sur la base desdites impédances, ladite mesure de synchronisme reflétant un synchronisme entre les mouvements de plan valvulaire respectivement du côté droit et du côté gauche du coeur pendant lesdites séances de mesure.

4. Dispositif médical implantable selon la revendication 3, dans lequel ledit circuit de détermination de paramètre hémodynamique (152) est adapté pour déterminer un synchronisme entre une fermeture de la valvule aortique et de la valvule pulmonaire et/ou pour déterminer un synchronisme entre une ouverture de la valvule aortique et de la valvule pulmonaire.

5. Dispositif médical implantable selon la revendication 3 ou 4, dans lequel ledit circuit de détermination de paramètres hémodynamiques (152) est adapté pour déterminer un synchronisme entre une fermeture de la valvule mitrale et respectivement de la valvule tricuspide, sur la base desdites impédances.

6. Dispositif médical implantable selon les revendications 3 à 5, dans lequel ledit circuit de détermination de délai AV et/ou VV est adapté pour
lancer une procédure d'optimisation, dans lequel ledit générateur d'impulsions de stimulation est commandé pour, sur la base de ladite mesure de synchronisme, ajuster de manière itérative un délai AV et/ou W présent pour identifier un délai AV et/ou VV qui provoque des mouvements de plan valvulaire essentiellement synchronisés respectivement du côté droit et du côté gauche du coeur, pendant un cycle cardiaque.

7. Dispositif médical implantable selon la revendication 6, dans lequel ledit circuit de détermination de délai AV et/ou VV (154) est adapté pour
lancer une procédure d'optimisation, dans lequel ledit générateur d'impulsions de stimulation (126) est commandé pour, sur la base dudit synchronisme entre une fermeture de la valvule aortique et de la valvule pulmonaire et/ou dudit synchronisme entre une ouverture de la valvule aortique et de la valvule pulmonaire, ajuster de manière itérative un délai AV et/ou VV présent pour identifier un délai AV et/ou VV qui provoque une fermeture et/ou une ouverture essentiellement synchronisée des valvules aortique et pulmonaire.

8. Dispositif médical implantable selon la revendication 6 ou 7, dans lequel ledit circuit de détermination de délai AV et/ou W (154) est adapté pour
lancer une procédure d'optimisation, dans lequel ledit générateur d'impulsions de stimulation (126) est commandé pour, sur la base dudit synchronisme entre une fermeture de la valvule mitrale et de la valvule tricuspide, ajuster de manière itérative un délai AV et/ou W présent pour identifier un délai AV et/ou W qui provoque une fermeture essentiellement synchronisée des valvules mitrale et tricuspide.

9. Dispositif médical implantable selon les revendications 1 à 8, dans lequel ledit circuit de mesure d'impédance (146) est adapté pour déterminer une impédance maximale et/ou minimale de chaque impédance respective pour chaque cycle cardiaque.

10. Dispositif médical implantable selon les revendications 1 à 9, dans lequel ledit circuit de mesure d'impédance (146) est adapté pour déterminer une dérivée absolue maximale de chaque impédance respective pour chaque cycle cardiaque.

11. Dispositif médical implantable selon les revendications 1 à 10, dans lequel
ledit circuit de mesure d'impédance (146) est adapté pour effectuer lesdites séances de mesure d'impédance pendant des cycles cardiaques successifs, dans lequel des signaux d'impédance reflétant des mouvements de plan valvulaire pendant lesdits cycles cardiaques successifs sont obtenus.

12. Dispositif médical implantable selon les revendications 1 à 8, dans lequel ladite au moins une électrode de plan valvulaire (26, 36 ; 55 ; 65, 67 ; 77 ; 85 ; 105, 112) est placée au niveau de l'endocarde.

13. Dispositif médical implantable selon les revendications 1 à 12, dans lequel ladite au moins une électrode de plan valvulaire (45 ; 75 ; 87 ; 95, 97 ; 111) est placée au niveau de l'épicarde.

14. Dispositif médical implantable selon les revendications 2 à 13, dans lequel ladite première électrode de plan valvulaire (26, 36 ; 55 ; 65, 67 ; 77 ; 85 ; 105, 112) est placée au niveau de l'endocarde dans l'oreillette droite, ou dans l'oreillette gauche, ou dans le ventricule gauche, ou dans le ventricule droit, ou au niveau de l'épicarde et dans lequel ladite seconde électrode de plan valvulaire (45 ; 75 ; 87 ; 95, 97 ; 111) est placée au niveau de l'endocarde dans l'oreillette droite, ou dans l'oreillette gauche, ou dans le ventricule gauche, ou dans le ventricule droit ou au niveau de l'épicarde.
